(19) **Europäisches Patentamt**
**European Patent Office**
**Office européen des brevets**

(11) **EP 2 318 835 B1**

(12) **EUROPEAN PATENT SPECIFICATION**

(45) Date of publication and mention
of the grant of the patent:
**12.04.2017 Bulletin 2017/15**

(21) Application number: **09800667.9**

(22) Date of filing: **22.07.2009**

(51) Int Cl.:
*G01N 33/52* *(2006.01)*  *G01N 33/50* *(2006.01)*

(86) International application number:
**PCT/US2009/004240**

(87) International publication number:
**WO 2010/011299 (28.01.2010 Gazette 2010/04)**

(54) **NANOSTRUCTURED OPTICAL FIBER ILLUMINATION SYSTEMS AND METHODS FOR BIOLOGICAL APPLICATIONS**

NANOSTRUKTURIERTE GLASFASERBELEUCHTUNGSSYSTEME UND VERFAHREN ZUR BIOLOGISCHEN ANWENDUNG

SYSTÈMES D'ÉCLAIREMENT À FIBRES OPTIQUES NANOSTRUCTURÉES ET PROCÉDÉS POUR DES APPLICATIONS BIOLOGIQUES

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **25.07.2008 US 137009 P**

(43) Date of publication of application:
**11.05.2011 Bulletin 2011/19**

(73) Proprietor: **Corning Incorporated**
**Corning, NY 14831 (US)**

(72) Inventors:
• **BICKHAM, Scott, R.**
**Corning, NY 14830 (US)**
• **DONG, Lonying**
**Elmira, NY 14903 (US)**
• **FEWKES, Edward, J.**
**Horseheads, NY 14845 (US)**
• **LOGUNOV, Stephan, L.**
**Corning, NY 14830 (US)**
• **WINNINGHAM, Michael, J.**
**Big Flats, NY 14814 (US)**

(74) Representative: **Greene, Simon Kenneth**
**Elkington and Fife LLP**
**Prospect House**
**8 Pembroke Road**
**Sevenoaks, Kent TN13 1XR (GB)**

(56) References cited:
**DE-A1- 3 600 635**     **DE-A1- 4 416 069**
**US-A1- 2005 053 266**     **US-A1- 2005 137 657**
**US-A1- 2005 244 952**     **US-A1- 2007 104 437**
**US-B1- 6 850 637**

## Description

## BACKGROUND OF THE INVENTION

## FIELD OF THE INVENTION

The present invention relates generally to nanostructured optical fibers, and in particular to illumination systems and methods that employ nanostructured fibers for biological applications.

## TECHNICAL BACKGROUND

Optical fibers are used for a variety of applications where light needs to be delivered from a light source to a remote location. Optical telecommunication systems, for example, rely on a network of optical fibers to transmit light from a central office to system end-users, e.g., in so-called "fiber-to-the-X" or "FTTX" systems, where "X" stands for the end-location of the fiber (e.g., "H" for "home," "C" for "curb," etc.).

Telecommunication optical fibers are designed to operate at near-infrared wavelengths in the range from 800 nm to 1675 nm where there are only relatively low levels of attenuation due to absorption and scattering. This allows most of the light inputted into one end of the fiber to exit the opposite end of the fiber with only insubstantial amounts exiting peripherally through the sides of the fiber.

More recently, there has been a growing need to have optical fibers that are less sensitive to bending than conventional fibers. This is because more and more telecommunication systems are being deployed in configurations that require the optical fiber to have strong bends. This need has lead to the development of so-called "nanostructured" optical fibers that utilize a ring of small non-periodically disposed voids that surround the core region. The air line ring serves to increase the bend insensitivity-that is to say, the fiber can have a smaller bend radius without suffering a significant change in the attenuation of the optical signal passing therethrough.

Because optical fibers are typically designed to efficiently deliver light from one end to the other over long distances, they are not typically considered well-suited for use in forming an extended illumination source because very little light escapes from the sides of the have a smaller bend radius without suffering a significant change in the attenuation of the optical signal passing therethrough.

Because optical fibers are typically designed to efficiently deliver light from one end to the other over long distances, they are not typically considered well-suited for use in forming an extended illumination source because very little light escapes from the sides of the typical fiber. Yet, there are a number of applications such as biological applications, including bacteria growth and the production of photobioenergy and biomass fuels, where select amounts of light needs to be provided in an efficient manner to remote growth areas such as photobioreactors. In particular, there is an urgent need to develop processes that convert light energy into high-value biomass-based fuels that are high-density and that can burn clean so that they can be used in internal combustion engines. Large scale production of biofuels will require increasingly more efficient reactors and light delivery methods. These needs can be fulfilled only if there are efficient light sources to deliver the light to the biological material.

Thus, it would be beneficial to have illumination systems and methods that exploit the ability of optical fibers to efficiently deliver light to remote locations if the fibers could also be adapted to form an extended light source.

DE 44 16 069 describes a method and apparatus for illuminating media for cultivation of phototropic organisms using optical fibers.

US2007/0104437 describes a microstructured optical fiber with voids in the cladding region.

US 2005/0137657 describes an optical device with a region wound in a coil which emits light.

## SUMMARY OF THE INVENTION

A first aspect of the invention is an illumination system for a biological growth system according to claim 1.

A second aspect of the invention is a biological growth system. The system includes a biological chamber with an interior configured to contain biological material. The system also includes a light source that generates light having a wavelength to which the biological material is sensitive. The system further includes at least one nanostructured optical fiber having a central axis, an outer surface and an end optically coupled to the light source. The fiber is configured to have a plurality of bends formed therein so as to scatter guided light material. The method includes forming, in at least one nanostructured optical fiber having a central axis and an outer surface, a plurality of bends configured to substantially increase Rayleigh scattering in the at least one fiber, the plurality of bends forming a light-source fiber portion of the at least one fiber. The method also includes disposing the light-source fiber portion in the biological chamber interior and inputting light into the at least one fiber and Rayleigh-scattering a portion of the light away from the central axis and through the outer surface, thereby emitting substantially uniform radiation from the light-source fiber portion. The substantially uniform radiation includes a wavelength to which the biological material is sensitive.

Additional features and advantages of the invention will be set forth in the detailed description which follows, and in part will be readily apparent to those skilled in the art from that description or recognized by practicing the invention as described herein, including the detailed description which follows, the claims, as well as the appended drawings.

It is to be understood that both the foregoing general description and the following detailed description present embodiments of the invention, and are intended to provide an overview or framework for understanding the nature and character of the invention as it is claimed. The accompanying drawings are included to provide a further understanding of the invention, and are incorporated into and constitute a part of this specification. The drawings illustrate various embodiments of the invention and together with the description serve to explain the principles and operations of the invention.

## BRIEF DESCRIPTION OF THE DRAWINGS

FIG. 1 is a schematic side view of a section of an example embodiment of a bend-insensitive optical fiber in the form of a nanostructure optical fiber;

FIG. 2 is a schematic cross-section of the optical fiber of FIG. 1 as viewed along the direction 2-2;

FIG. 3A is a relative refractive index plot versus fiber radius for an example multimode nanostructured fiber as shown in FIG. 2 and that includes an inner annular cladding region between the core and the nanostructured region;

FIG. 3B is a plot similar to FIG. 3A but for an example multimode nanostructured fiber wherein the nanostructured region immediately surrounds the core;

FIG. 4 is a schematic diagram of an example embodiment of a biological growth system that includes an illumination system in combination with a biological chamber;

FIG. 5 is a close-up view of the multimode nanostructured fiber used in the illumination system of FIG. 4, wherein the input fiber portion (12A) is coupled to another section of nanostructure optical fiber optical coupling device;

FIG. 6 is close-up view of similar to FIG. 5, illustrating an example embodiment wherein the input fiber portion is formed by a different type of optical fiber (e.g., a non-nanostructure optical fiber) and is optically coupled to a multimode nanostructured fiber making up the light-source fiber portion;

FIG. 7 is a plot of the loss (dB/km) versus wavelength (nm) for a typical telecommunications optical fiber, illustrating the very large losses in the visible wavelength range as compared to the near-IR wavelengths of 800 nm and above;

FIG. 8 is a close-up view of a bend in the multimode nanostructured fiber as formed in the light-source fiber portion of the fiber, illustrating the bend radius $R_B$ and the radiated light caused by the bend;

FIG. 9 is a plot of intensity I (normalized units) as a function of distance D (meters) along a fiber 12 of length L illustrating how the amount (intensity) of radiated light diminishes as function of distance along the fiber and how counter-wrapping the fiber creates substantially uniform radiated light over the distance

L;

FIG. 10 is a plot of the relative Intensity vs. Distance D (meters) for an example embodiment of the light-source fiber portion of the fiber illumination system that includes four counter-wound layers of multi-mode nanostructured fiber to create substantially uniform radiated light;

FIG. 11A is a close-up view of a portion of the illumination system of FIG. 4, illustrating an example embodiment of the light-source fiber portion of the fiber that includes two counter-wound fibers;

FIG. 11B is similar to FIG. 11A, illustrating an example embodiment of the light-source fiber portion of the fiber wherein the same fiber is counter-wound;

FIG. 11C is similar to FIG. 11B, illustrating an example embodiment of the light-source fiber portion of the fiber with multiple counter-windings that are relatively tight and angled in opposite directions;

FIG. 11D is similar to FIG. 11C, and illustrates an example embodiment of the light-source fiber portion of the fiber with even more counter-windings;

FIG. 12 illustrates an example embodiment of a portion of the illumination system of FIG. 4 wherein multiple fibers are arranged in a sequence of looped sections to form an extended light source;

FIG. 13 illustrates an example embodiment of the front portion of the illumination system wherein the light source and optical coupling system are configured to couple light into the respective input ends of multiple fibers;

FIG. 14 illustrates an example embodiment of illumination system as used in combination with a biological chamber in the form of a flask;

FIG. 15 illustrates an example embodiment (top-down view) wherein the light-source fiber portion of illumination system is configured for use in rectangular-cross-section biological chamber;

FIG. 16 illustrates an example embodiment (top-down view) wherein the light-source fiber portion of illumination system is configured for use in rectangular-cross-section biological chamber;

FIG. 17A is a plot of biomass (expressed as cell density) vs. days of inoculation growth for cyanobacteria that includes a test group illuminated with the illumination system of the present invention of FIG. 4 and FIG. 14, a control group illuminated with a fluorescent lamp of equal photosynthetic photon flux (PPF);

FIG. 17B is a plot of biomass (expressed as cell density) vs. days of inoculation growth for cyanobacteria that includes a test group illuminated with the illumination system of FIG. 4 and FIG. 14, a control group illuminated through the tip if the fiber with same light power; and

FIG. 18 is a plot of fiber loss vs. bending diameter, for the exemplary fiber diameters.

Additional features and advantages of the invention will be set forth in the detailed description which follows and will be apparent to those skilled in the art from the description or recognized by practicing the invention as described in the following description together with the claims and appended drawings.

## DETAILED DESCRIPTION

Reference is now made in detail to the present preferred embodiments of the invention, examples of which are illustrated in the accompanying drawings. Whenever possible, like or similar reference numerals are used throughout the drawings to refer to like or similar parts. It should be understood that the embodiments disclosed herein are merely examples, each incorporating certain benefits of the present invention.

Various modifications and alterations may be made to the following examples within the scope of the present invention, and aspects of the different examples may be mixed in different ways to achieve yet further examples. Accordingly, the true scope of the invention is to be understood from the entirety of the present disclosure, in view of but not limited to the embodiments described herein.

### Definitions

Terms such as "horizontal," "vertical," "front," "back," etc., and the use of Cartesian Coordinates are for the sake of reference in the drawings and for ease of description and are not intended to be strictly limiting either in the description or in the claims as to an absolute orientation and/or direction.

In the description of the invention below, the following terms and phrases are used in connection nanostructure optical fibers.

The "refractive index profile" is the relationship between the refractive index or the relative refractive index and the waveguide (fiber) radius.

The "relative refractive index percent" is defined as

$$\Delta(r)\% = 100 \times [n(r)^2 - n_{REF}^2)]/2n(r)^2,$$

where $n(r)$ is the refractive index at radius r, unless otherwise specified. The relative refractive index percent is measured at 850 nm unless otherwise specified.

In one aspect, the reference index $n_{REF}$ is the refractive index at the core/clad interface. In another aspect, $n_{REF}$ is the average refractive index of the outer annular portion of the cladding, which can be calculated, for example, by taking "N" index measurements ($n_{C1}$, $n_{C2}$, ... $n_{CN}$) in the outer annular portion of the cladding, and calculating the average refractive index by:

$$n_C = (1/N) \sum_{i=1}^{N} n_{Ci}.$$

As used herein, the relative refractive index is represented by $\Delta$ and its values are given in units of "%", unless otherwise specified. In cases where the refractive index of a region is less than the reference index $n_{REF}$, the relative index percent is negative and is referred to as having a depressed region or depressed-index, and the minimum relative refractive index is calculated at the point at which the relative index is most negative unless otherwise specified. In cases where the refractive index of a region is greater than the reference index $n_{REF}$, the relative index percent is positive and the region can be said to be raised or to have a positive index.

An "updopant" is herein considered to be a dopant which has a propensity to raise the refractive index relative to pure undoped $SiO_2$. A "downdopant" is herein considered to be a dopant which has a propensity to lower the refractive index relative to pure undoped $SiO_2$. An updopant may be present in a region of an optical fiber having a negative relative refractive index when accompanied by one or more other dopants which are not updopants. Likewise, one or more other dopants which are not updopants may be present in a region of an optical fiber having a positive relative refractive index. A downdopant may be present in a region of an optical fiber having a positive relative refractive index when accompanied by one or more other dopants which are not downdopants.

Likewise, one or more other dopants which are not downdopants may be present in a region of an optical fiber having a negative relative refractive index.

The term "$\alpha$-profile" or "alpha profile" refers to a relative refractive index profile, expressed in terms of $\Delta(r)$ which is in units of "%", where r is radius, which follows the equation,

$$\Delta(r) = \Delta(r_o)(1 - [|r-r_o| / (r_1-r_o)]^{\alpha}),$$

where $r_0$ is the point at which $\Delta(r)$ is maximum, $r_1$ is the point at which $\Delta(r)\%$ is zero, and r is in the range $r_i \leq r \leq r_f$, where $\Delta$ is defined above, $r_i$ is the initial point of the $\alpha$-profile, $r_f$ is the final point of the $\alpha$-profile, and $\alpha$ is an exponent which is a real number.

As used herein, the term "parabolic" therefore includes substantially parabolically shaped refractive index profiles which may vary slightly from an $\alpha$ value of 2.0 at one or more points in the core, as well as profiles with minor variations and/or a centerline dip.

Macrobend performance of the nanostructure fiber considered herein was determined according to TIA/EIA-455-62-A FOTP-62 (IEC-60793-1-47) by wrapping 1 turn around a either a 10 mm or 20 mm diameter mandrel (the "1x10 mm diameter macrobend loss" or the "1x20 mm diameter macrobend loss") and measuring the increase in attenuation due to the bending using an overfilled launch condition. Bandwidth was measured according to TIA/EIA-455-204 FOTP-204 with overfilled launch.

*Bend insensitive optical fibers*

Example embodiments of the present invention make use of bend-insensitive or bend-resistant fibers such as those in the form of so-called "nanostructured" or "holey" optical fibers. Some embodiments of the present invention make use of bend-insensitive or bend-resistant fibers wherein all or a portion of the cladding is comprised of fluorine-doped silica.

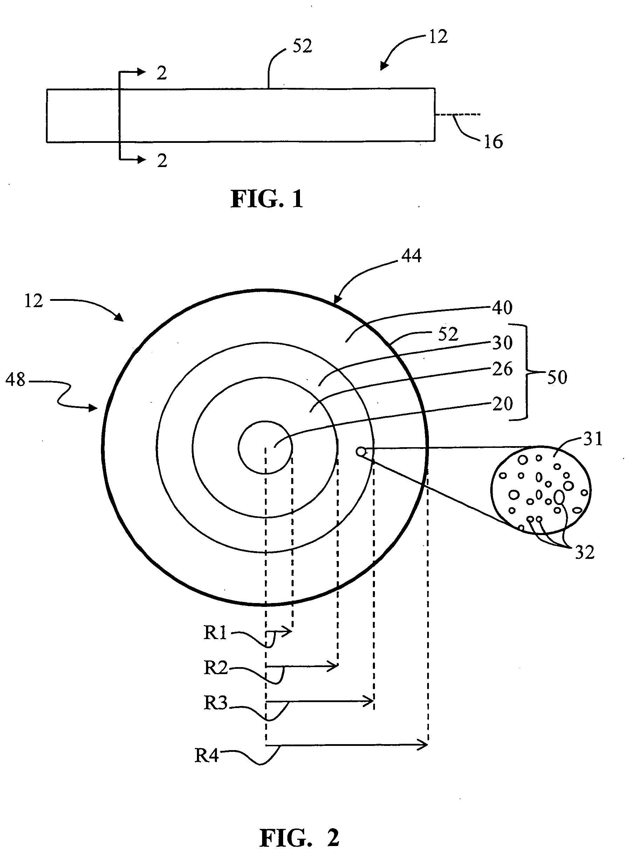

FIG. 1 is a schematic side view of a section of an example embodiment of a bend-insensitive fiber in the form of a multimode nanostructure optical fiber (hereinafter "fiber") 12 having a central axis ("centerline") 16. FIG. 2 is a schematic cross-section of nanostructure fiber 12 as viewed along the direction 2-2 in FIG. 1. Fiber 12 can be, for example, any one of the various types of nanostructure optical fibers, such as any of the so-called "holey" fibers, or those described in the above-mentioned Corning nanostructure fiber patents and patent applications. For the purposes of the present invention, a "bend-insensitive fiber" includes nanostructure fibers that make use of periodic or non-periodic nanostructures or voids. In an example embodiment, fiber 12 includes a core region ("core") 20, an inner annular cladding region 26, an annular nanostructure region 30 surrounding the inner annular cladding region, and an outer annular cladding region 40 ("cladding") surrounding the annular nanostructure region. Inner cladding region 26, nanostructure region 30 and outer cladding region 40 constitute a "cladding structure" 50 that has an outer surface 52.

An optional layer 44 surrounds outer cladding region 40. In an example embodiment, layer 44 is a coating comprising a low modulus primary coating and a high modulus secondary coating. In some embodiments, layer 44 comprises a polymer coating called "holey" fibers, or those described in the above-mentioned Corning nanostructure fiber patents and patent applications. For the purposes of the present invention, a "bend-insensitive fiber" includes nanostructure fibers that make use of periodic or non-periodic nanostructures or voids. In an example embodiment, fiber 12 includes a core region ("core") 20, an inner annular cladding region 26, an annular nanostructure region 30 surrounding the inner annular cladding region, and an outer annular cladding region 40 ("cladding") surrounding the annular nanostructure region. Inner cladding region 26, nanostructure region 30 and outer cladding region 40 constitute a "cladding structure" 50 that has an outer surface 52.

An optional layer 44 surrounds outer cladding region 40. In an example embodiment, layer 44 is a coating comprising a low modulus primary coating and a high modulus secondary coating. In some embodiments, layer 44 comprises a polymer coating such as an acrylate-based polymer. In some embodiments, the coating has a constant diameter along the length of the fiber.

In other example embodiments described below, layer 44 is designed to enhance the distribution and/or the nature of "radiated light" that passes from core 20 through cladding structure 50. Outer cladding region 40 (or optional layer 44) represents the "sides" 48 of fiber 12 through which light traveling in the fiber is made to exit via scattering, as described below.

A protective cover or sheath (not shown) optionally covers outer cladding 40. In an example embodiment, nanostructure region 30 comprises a glass matrix ("glass") 31 having formed therein non-periodically disposed holes (also called "voids" or "nanostructures") 32, such as the example voids shown in detail in the magnified inset of FIG. 2. In another example embodiment, voids 32 may be periodically disposed, such as in a photonic crystal optical fiber, wherein the voids typically have diameters between about $1 \times 10^{-6}$ m and $1 \times 10^{-5}$ m. Voids 32 may also be non-periodically or randomly disposed. In an example embodiment, glass 31 is fluorine-doped while in another example embodiment the glass is undoped pure silica. By "non-periodically disposed" or "non-periodic distribution," it is meant that when one takes a cross-section of the optical fiber (such as shown in FIG. 2), the voids 32 are randomly or non-periodically distributed across a portion of the fiber. Similar cross sections taken at different points along the length of the fiber will reveal different cross-sectional hole patterns, i.e., various cross sections will have different hole patterns, wherein the distributions of holes and sizes of holes do not match. That is, the holes or holes are non-periodic, i.e., they are not periodically disposed within the fiber structure. These holes are stretched (elongated) along the length (i.e. parallel to the longitudinal axis) of the optical fiber, but do not extend the entire length of the entire fiber for typical lengths of transmission fiber. While not wishing to be bound by theory, it is believed that the holes extend less than a few meters, and in many cases less than 1 meter along the length of the fiber.

Fiber 12 as used herein in the illumination system discussed below can be made by methods which utilize preform consolidation conditions which are effective to result in a significant amount of gases being trapped in the consolidated glass blank, thereby causing the formation of holes in the consolidated glass optical fiber preform. Rather than taking steps to remove these holes, the resultant preform is used to form an optical fiber with airlines, or nanostructures, therein.

As used herein, the diameter of a hole is the longest line segment whose endpoints are disposed on the silica internal surface defining the hole when the optical fiber is viewed in perpendicular cross-section transverse to the longitudinal axis of the fiber. Methods of making such optical fibers with holes is described in U.S. Patent Application Serial No. 11/583,098.

In some embodiments of fiber 12, core 20 comprises silica doped with germanium, i.e., germania-doped silica. Dopants other than germanium, singly or in combination, may be employed within the core, and particularly at or near centerline **16**, of the optical fiber to obtain the desired refractive index and density. In some embodiments, the refractive index profile of the optical fiber disclosed herein is non-negative from the centerline to the outer radius of the core. In some embodiments, the optical fiber contains no index-decreasing dopants in the core.

Fiber **12** may include a fluorinated cladding structure **50**, but it is not needed if the fibers are to be used as short-length light pipes. A pure silica core **20** is one of the desired properties of fiber **12**, but a preferred attribute of the fiber is its ability to scatter light out of the fiber in the desired spectral range to which biological material is sensitive. The amount of the loss via scattering can be increased by changing properties of the glass in the fiber.

In some examples of fiber **12** as used herein, core **20** is a graded-index core, and preferably, the refractive index profile of the core has a parabolic (or substantially parabolic) shape; for example, in some embodiments, the refractive index profile of core **20** has an $\alpha$-shape with an $\alpha$ value of about 2, preferably between 1.8 and 2.3, as measured at 850 nm; in some embodiments, the refractive index of the core may have a centerline dip, wherein the maximum refractive index of the core, and the maximum refractive index of the entire optical fiber, is located a small distance away from centerline **16**, but in other embodiments the refractive index of the core has no centerline dip, and the maximum refractive index of the core, and the maximum refractive index of the entire optical fiber, is located at the centerline.

One or more portions of cladding structure **50** may comprise a cladding material deposited, for example during a laydown process, or that was provided in the form of a jacketing, such as a tube in a rod-in-tube optical preform arrangement, or a combination of deposited material and a jacket.

In an example embodiment, fiber **12** has a silica-based core and cladding. In some embodiments, the cladding has an outer diameter 2 times Rmax, e.g., of about 125 $\mu$m. Preferably, the outer diameter of the cladding has a constant diameter along the length of fiber **12**. In some embodiments, the refractive index of fiber **12** has radial symmetry. Preferably, the outer diameter (2R1) of core **20** has a constant diameter along the length of the fiber.

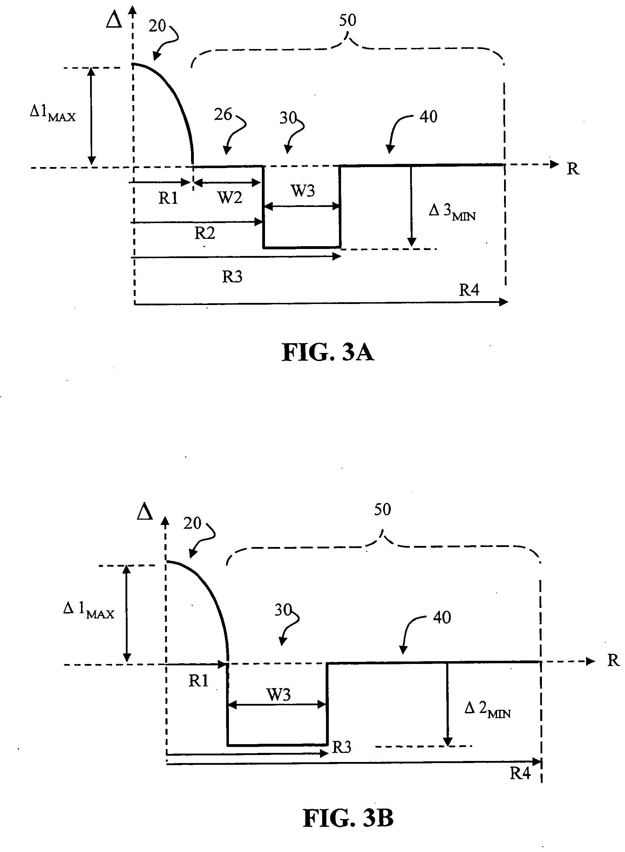

**FIG. 3A** is a plot of the relative refractive index $\Delta$ versus fiber radius for an example fiber **12** as shown in **FIG. 2**. Core **20** extends radially outwardly from the centerline to a core outer radius, **R1**, and has a relative refractive index profile $\Delta_1(r)$ with a maximum relative refractive index percent $\Delta_{1MAX}$. In the first aspect, the reference index $n_{REF}$ is the refractive index at the core/clad interface, i.e. at radius **R1**. Inner annular cladding region **26** has a refractive index profile $\Delta2(r)$ with a maximum

relative refractive index $\Delta2_{MAX}$, and a minimum relative refractive index $\Delta2_{MIN}$, where in some embodiments $\Delta2_{MAX} = \Delta2_{MIN}$. Nanostructure region **30** has a refractive index profile $\Delta3(r)$ with a minimum relative refractive index $\Delta3_{MIN}$. The outer annular cladding region **40** has a refractive index profile $\Delta4(r)$ with a maximum relative refractive index $\Delta4_{MAX}$, and a minimum relative refractive index $\Delta4_{MIN}$, where in some embodiments $\Delta4_{MAX} = \Delta4_{MIN}$. Also, $\Delta1_{MAX} > \Delta2_{MAX} \geq \Delta2_{MIN} > \Delta3_{MIN}$, and $\Delta1_{MAX} > \Delta4_{MAX} \geq \Delta4_{MIN} > \Delta3_{MIN}$.

In some embodiments, inner annular cladding region **26** has a substantially constant refractive index profile, as shown in **FIG. 3A**, with a constant $\Delta2(r)$. In some of these embodiments, $\Delta2(r) = 0\%$. In some embodiments, the outer annular cladding region **40** has a substantially constant refractive index profile, as shown in **FIG. 3A** with a constant $\Delta4(r)$. In some of these embodiments, $\Delta4(r) = 0\%$. The core **20** has an entirely positive refractive index profile, where $\Delta1(r) > 0\%$. In some embodiments, the inner annular cladding region **26** has a relative refractive index profile $\Delta2(r)$ having a maximum absolute magnitude less than 0.05%, and $\Delta2_{MAX} < 0.05\%$ and $\Delta2_{MIN} > -0.05\%$, and nanostructure region **30** begins where the relative refractive index of the cladding first reaches a value of less than -0.05%, going radially outwardly from the centerline.

In some embodiments, the outer annular cladding region **40** has a relative refractive index profile $\Delta4(r)$ having a maximum absolute magnitude less than 0.05%, and $\Delta4_{MAX} < 0.05\%$ and $\Delta4_{MIN} > -0.05\%$, and nanostructure region **30** ends where the relative refractive index of the cladding first reaches a value of greater than -0.05%, going radially outwardly from the radius where $\Delta3MIN$ is found. In some embodiments, the inner annular portion **30** comprises pure silica.

In some embodiments, the outer annular cladding **40** comprises pure silica. In some embodiments, nanostructure region **30** comprises pure silica comprising a plurality of holes **32**. Preferably, the minimum relative refractive index, or average effective relative refractive index, such as taking into account the presence of any holes, of nanostructure region **30** is preferably less than -0.1 %. Holes **32** can contain one or more gases, such as argon, nitrogen, or oxygen, or the holes can contain a vacuum with substantially no gas; regardless of the presence or absence of any gas, the refractive index in nanostructure region **30** is lowered due to the presence of holes **32**. Holes **32** can be randomly or non-periodically disposed in nanostructure region **30**, and in other embodiments, the holes are disposed periodically therein.

In some embodiments, the plurality of holes **32** comprises a plurality of non-periodically disposed holes and a plurality of periodically disposed holes. Alternatively, or in addition, the depressed index in annular portion **50** can also be provided by downdoping nanostructure region **30** (such as with fluorine) or updoping one or more portions of the cladding and/or the core, wherein nanostructure region **30** is, for example, pure silica or silica which is not doped as heavily as the inner annular portion **30**.

In one set of embodiments, fiber **12** comprises a graded-index, preferably parabolic (substantially parabolic), glass core **20** and glass cladding structure **50** as depicted in **FIG. 2** wherein core **20** ends at a radius **R1**, which marks the end of the graded index core or parabolic shape. Core **20** is surrounded by and in direct contact with the inner annular cladding **26**, which has a substantially constant refractive index profile $\Delta2(r)$. The inner annular cladding **26** is surrounded by and in direct contact with nanostructure region **30**, and this region in turn is surrounded by and in direct contact with the outer annular cladding **40**, which has a substantially constant refractive index profile $\Delta4(r)$.

In example embodiments, core **20** comprises germania doped silica, inner annular region **26** comprises pure silica, and the outer annular region **40** comprises pure silica; in some of these embodiments, nanostructure region **30** comprises a plurality of holes **32** in pure silica; and in yet others of these embodiments, nanostructure region **30** comprises a plurality of holes **32** in fluorine-doped silica.

In embodiments where the inner annular cladding **26** comprises pure silica and the nanostructure region **30** comprises pure silica with a plurality of holes **32**, the nanostructure region starts at the innermost radius of the innermost hole. In embodiments where the outer annular cladding **40** comprises pure silica, and nanostructure region **30** comprises pure silica with a plurality of holes **32**, the nanostructure region ends at the outermost radius of the outermost hole.

In an example embodiment, inner annular cladding **26** has a radial width **W2** of greater than 0.5 micron and less than 5 microns. In some embodiments, the minimum relative refractive index of nanostructure region **30**, $\Delta3MIN$, is less than -0.2%; in other embodiments, $\Delta3MIN$ is less than -0.3%; in still other embodiments, $\Delta3MIN$ is less than - 0.4%; in yet other embodiments, $\Delta3MIN$ is less than -0.6%.

$\Delta1_{MAX}$ is preferably less than or equal to 2.2%, more preferably less than or equal to 1.2%.

The numerical aperture (NA) of fiber **12** is preferably greater than the NA of a light source (e.g., light source **150** introduced and discussed below) directing light into the fiber.

In some embodiments, the core outer radius **R1** is preferably not less than 24 $\mu$m and not more than 50 $\mu$m, i.e. the core diameter is between about 48 and 100 $\mu$m. In other embodiments, R1 > 24 microns; in still other embodiments, R1 > 30 microns; in yet other embodiments, R1 > 40 microns.

In some embodiments, $|\Delta_2(r)| < 0.025\%$ for more than 50% of the radial width of the annular inner portion **26**, and in other embodiments $|\Delta_2(r)| < 0.01\%$ for more than 50% of the radial width of region **26**. The depressed-index annular portion **30** begins where the relative refractive index of the cladding first reaches a value of less than -0.05%, going radially outwardly from the centerline. In some embodiments, the outer annular portion **40** has a relative refractive index profile $\Delta4(r)$ having a maximum absolute magnitude less than 0.05%, and $\Delta4_{MAX} < 0.05\%$ and $\Delta4_{MIN} > -0.05\%$, and the depressed-index annular portion **30** ends where the relative refractive index of the cladding first reaches a value of greater than - 0.05%, going radially outwardly from the radius where $\Delta3MIN$ is found.

The width $W_3$ of nanostructured region **30** is R3-R2 and its midpoint $R_{3MID}$ is (R2+ R3)/2. In some embodiments, $W_3$ is greater than 1 and less than 20 $\mu$m. In other embodiments, $W_3$ is greater than 2 $\mu$m and less than 20 $\mu$m. In other embodiments, $W_3$ is greater than 2 $\mu$m and less than 12 $\mu$m.

Cladding structure **40** extends to a radius **R4**, which is also the outermost periphery of the glass part of the optical fiber. In some embodiments, R4 > 50 $\mu$m; in other embodiments, R4 > 60 $\mu$m, and in some embodiments, R4 > 70 $\mu$m.

In some embodiments, $\Delta_{3MIN}$ is less than (i.e. more negative than) -0.2%. In other embodiments, $\Delta_{3MIN}$ is less than -0.4%. In other embodiments, is less than -0.2% and greater than -3.0%.

**FIG. 3B** is a plot similar to **FIG. 3A** and showing an example embodiment wherein R2 = 0 so that there is no inner cladding region **26**, leaving nanostructure region **30** immediately adjacent core **20**. In the second aspect, $n_{REF}$ is the average refractive index of outer annular cladding **40**.

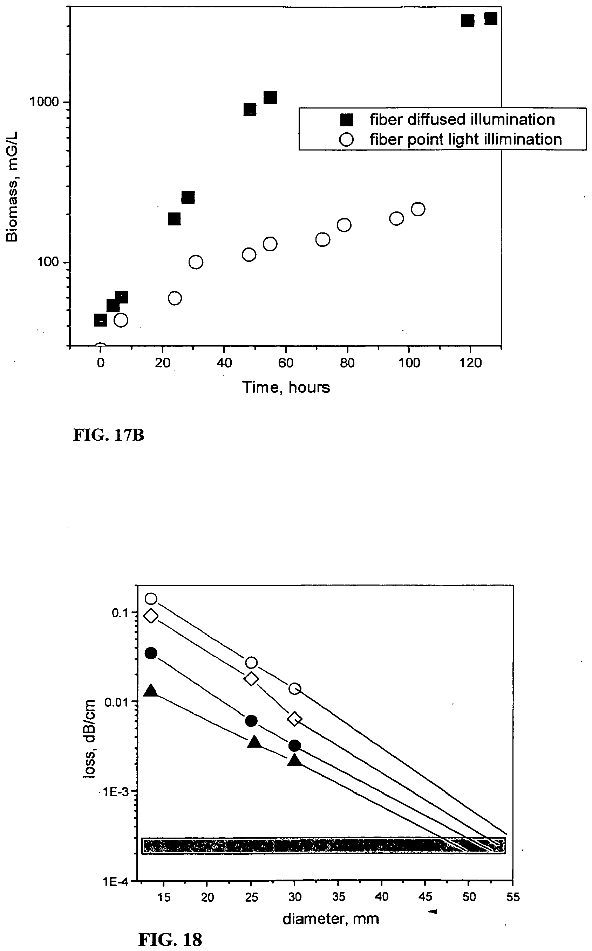

**FIG. 18** shows the measured macrobend losses at 530 nm as a function of bend diameter of four fiber embodiments of the present invention. The minimum bend diameter is defined as the bend diameter at which the bend loss for one turn at that diameter is equal to the scattering losses in the fiber at the operating wavelength. In some preferred embodiments, the 1x40 mm bending loss at the operating wavelength is less than the intrinsic scattering losses from the fiber at the operating wavelength. In other preferred embodiments, the 1x45 mm bending loss at the operating wavelength is less than the intrinsic scattering losses from the fiber at the operating wavelength. In other preferred embodiments, the 1x50 mm bending loss at the operating wavelength is less than the intrinsic scattering losses from the fiber at the operating wavelength. The operating wavelength is preferably less than 700 nm, more preferably less than 600 nm and even more preferably between 400 and 700 nm. Thus, we determined a minimum bending diameter of the fiber, such that the bending loss is less or comparable to intrinsic loss of the fiber.

### Illumination System

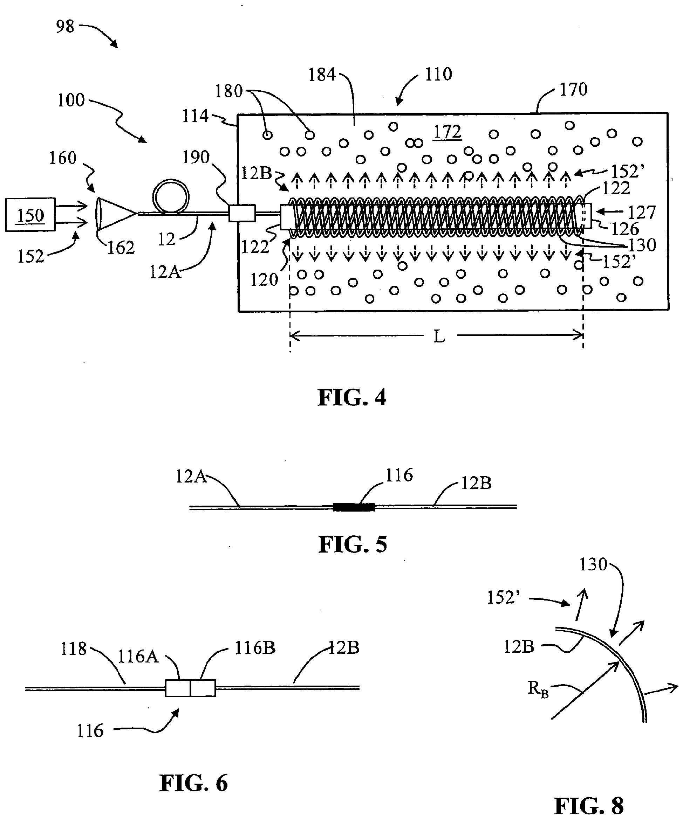

**FIG. 4** is a schematic diagram of an example embodiment of a biological growth system **98** that includes an illumination system **100** in combination with a biological chamber **110**. Illumination system **100** employs at least one fiber **12** as described above. In an example embodiment, the least one fiber **12** (referred below simply as "fiber **12**" for ease of discussion) is configured to include an optional first portion **12A** where the optical loss ("loss") in the fiber is not substantial. Fiber portion **12A** is used to convey light and is thus referred to as the "input fiber portion." Fiber **12** is also configured to have a second portion **12B** optically coupled with the input fiber portion wherein the loss in the second portion due to guided light being scattered out of "sides" **48** (i.e., out of cladding outer surface **52**). Fiber portion **12B** thus constitutes an extended light source of length **L** and is referred to as the "light-source fiber portion."

In an example embodiment, input fiber portion **12A** is straight or has a straight section, or includes gentle bends that do not induce substantially scattering. In another example embodiment, there is no input fiber portion **12A** and light is inputted directly into light-source fiber portion **12B**.

In yet another example embodiment, light-source fiber portion **12B** includes a coiled section having at least one and preferably multiple bends **130** that induce substantial amounts of Rayleigh scattering so that light is scattered out of sides **48**. In an example embodiment, the at least one bend is a "continuous" bend such as associated with a coil. In other example embodiments, the bends are constituted by a series of separate loops.

Fiber **12** includes an input end **12I** shown in **FIG. 4** as being at the end of input fiber portion **12A**. Input end **12I** can also be at the end of light-source fiber portion **12B** if there is no input fiber portion **12A**.

**FIG. 5** is a close-up view of fiber **12** illustrating an example embodiment of illumination system **100** wherein input fiber portion **12A** constitutes a first section of nanostructure fiber optically coupled to another section of nanostructure optical fiber making up light-source fiber portion **12B**. The coupling between the fiber sections is accomplished via an optical coupling device **116**.

**FIG. 6** is a another close-up view of similar to **FIG. 5**, illustrating an example embodiment of illumination system **100** wherein input fiber portion **12A** is formed by a different type of optical fiber (e.g., a non-nanostructure optical fiber) **118** optically coupled to light-source fiber portion **12B** via optical coupling device **116**. Optical coupling device can be, for example, a splice (e.g., fusion or mechanical), or respective optical connectors **116A** and **116B** as shown in **FIG. 6**.

With reference again to **FIG. 4**, in an example embodiment illumination system **100** includes a support structure **120** around which light-source fiber portion **12** is bent (e.g., coiled, as shown) to form one or more bends **130**. In an example embodiment, support structure **120** is a rod, as shown. In an example embodiment, support structure **120** comprises multiple sections.

An example support structure **120** such as the one shown in **FIG. 4** includes an outer surface **122** and first and second ends **124** and **126**. In an example embodiment, support structure **120** is hollow to reduce weight, and in a further example embodiment is made of a lightweight, inert material such as plastic, TYLON or PVC tubing. Hollow support structure **120** defines an interior **127**. In an example embodiment, support structure **120** is transparent to a wavelength of light conducted by light-source fiber portion **12B** so that the support structure does not substantially interfere with or otherwise substantially reduce the amount of light emitted by the light-source fiber portion. In another example embodiment, support structure **120** is reflective to a wavelength of light conducted by light-source fiber portion **12B** so that the support structure enhances the amount of light peripherally emitted by the light-source fiber portion.

In an example embodiment, support structure **120** is flexible and can be bent so that the light-source fiber portion **12B** can be formed into a number of different shapes (e.g., curved, circular, spiral, etc.). A number of support structure elements can also be combined as described below to form a wide range of different support structure geometries such as a grid, crosses, squares, rectangles, etc. Support structure **120** is preferably configured to be suitable for use with the particular configuration of the biological chamber **110** used.

In an example embodiment, light-source fiber portion **12B** is formed by winding (e.g., coiling) fiber **12** around support structure surface **122**. However, in other example embodiments, support structure **120** is not used or is not a part of the final illumination system **100**. Rather, bends **130** are first formed (e.g., using support structure **120**) and then fiber **12** treated so that bends **130** are maintained. For example, bends **130** may be formed by bending light-source fiber portion **12B** around support structure **120**, then using and adhesive or epoxy to fix bends **130** in place, and then removing the support structure. In another example embodiment, fiber **12** is wound around the inside of a hollow, transparent support structure **120** to form light-source fiber portion **12B**.

With continuing reference to **FIG. 4**, illumination system **100** further includes a light source **150** optically coupled to input end **12I** of fiber **12**. Light source **150** emits light **152**. Light **152** from light source **150** includes at least one wavelength to which the biological material to be illuminated is sensitive.

In an example embodiment, the optical coupling is accomplished using an optical coupling system **160**, such as may be comprised of one or more optical elements **162** arranged between light source **150** and fiber input end **12I**. In example embodiments, light source **150** comprises a laser, one or more light-emitting diodes, light bulbs, or is the sun-- in which case optical coupling system **160** is comprises one or more solar-collection elements (e.g., one or more mirrors) as optical element(s) **162**.

With continuing reference to **FIG. 4**, biological chamber **110** includes one or more walls **170** that define an interior **172**. In an example embodiment, interior **172** contains light-sensitive biological material **180**, such as algae (e.g., algae colonies, algae blooms) or bacteria (e.g., cyanobacteria). In an example embodiment, biological material **180** may be suspended in a support medium **184** such as water. At least a portion of light-source fiber portion **12B** is disposed within chamber interior **172** to illuminate the chamber interior and biological material **180** contained therein. In an example embodiment, fiber **12** is feed into chamber interior **172** via an opening **190**, which in an example embodiment is configured to be sealable to prevent the unwanted egress of biological material **180** and/or support medium **184** from chamber **110**.

In the operation of illumination system **100**, light source **150** is activated so that it generates light **152**. Light **152** is coupled into fiber **12** at input end **12I** via optical coupling system **160**. As discussed above, light **152** includes a wavelength to which biological material **180** is sensitive, and in a particular example embodiment the wavelength is one that causes the biological material to grow.

In an example embodiment, light **152** from light source **150** comprises light pulses generated in a manner to improve efficiency of cell growth in certain types of biological material **180** by avoiding photoinhibition.

Light **152** travels down input fiber portion **12A** and is substantially contained therein so that most of the input light is transmitted to light-source fiber portion **12B**. Scattering of light away from central axis **16** and out of outer surface **52** in light-source fiber portion **12B** generates substantially uniform illumination over the length of the light-source fiber portion, i.e., the light-source fiber portion serves as an extended light source that provides substantially uniform illumination in the form of emitted radiation **152'**. In an example embodiment, the predominant light-scattering mechanism in light-source fiber portion **12B** is Rayleigh scattering. In an example embodiment, a portion of emitted radiation **152'** is from bending loss, as described below.

### Forming substantially uniform radiation

While fiber **12** typically has relatively low attenuation from scattering at its intended near-IR wavelength range for telecommunication applications, the attenuation from scattering is much higher at the shorter visible wavelengths to which biological material **180** is most sensitive.

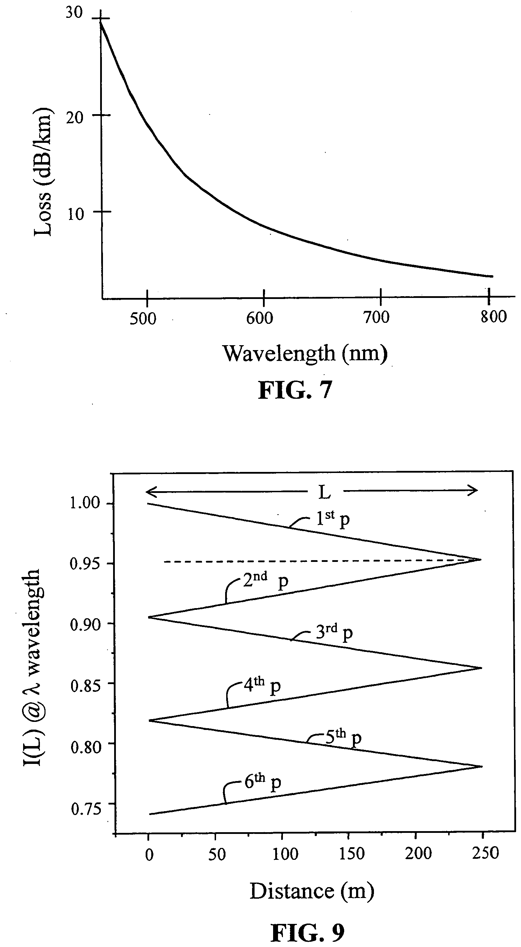

FIG. 7 is a plot of the attenuation (loss) in dB/km versus wavelength (nm) for a typical telecommunications optical fiber, which illustrates the very large losses in the visible wavelength range as compared to the near-IR wavelengths of 800 nm and above.

It is worth noting that even at the shorter visible wavelengths, fiber **12** remains substantially bend insensitive so that "hot spots" do not occur due to bending loss from bends **130**. In an example embodiment, the "bend insensitivity" manifests itself as a uniform loss at visible wavelengths, as opposed to fiber **12** having small bending loss at such wavelengths. Thus, in some cases a portion of emitted radiation **152'** is from bending loss while another portion (e.g., the remaining portion) of the emitted radiation is due to scattering loss. The important point here is that the bending loss, while not necessarily small, remains substantially uniform. Such uniform bending loss mitigates the creating of "hot-spots" that otherwise occur if the fiber is bend-sensitive, which leads to bend-dependent losses in the fiber that create substantial non-uniformity in emitted radiation **152'**.

With reference also to FIG. 8, bends **130** in light-source fiber portion **12B** have a bend radius $R_B$ such that the scattering of light **152** is substantially enhanced, and is on the order of about 10 to 20 dB/km at the visible wavelengths. Thus, when light **152** reaches light-source fiber portion **12B** and bends **130** therein, scattering causes a portion of the light to be scattered out of the fiber along the length of the fiber as radiated light **152'**. A portion of radiated light **152'** is then absorbed by biological material **180**, thereby enhancing the growth rate of the biological material.

As discussed above, in an example embodiment, nanostructure region **30** of fiber **12** has holes **32** on the order of 200 nm to 500 nm in diameter. The diameter of holes **32** can be controlled in the consolidation and draw processes when forming fiber **12**. The aforementioned dimensions of holes **32** are comparable to the desired wavelength range for photo biosynthesis, so that the bending-induced Rayleigh scattering will efficiently radiated light **152'**.

FIG. 9 is a plot of intensity I as a function of distance D (meters) along fiber **12** of length $L_F$. The plot illustrates how the amount (intensity) of radiated light **152'** diminishes as function of distance along the fiber for each length $L_F$. The plot of FIG. 9 indicates that the intensity I drops linearly by 5% over each pass of length $L_F$ through the fiber.

Note, however, that by looping fiber **12** back on itself (i.e.,"counter-winding" the fiber ) six times (thereby forming six fiber layers or "windings") to form light-source fiber portion **12B** of length **L**. Now, the light **152** effectively makes six passes over the same (folded) length **L** while emitting radiated light **152'**.

Consider, for example, the case for fiber **12** having two counter-winding so that there are two passes of the fiber over length **L**. The total intensity is the sum of the two intensity curves for each pass. At the input end (D=0), the sum of the intensities is 100 + 90 = 190. At the opposite end D = L, the sum of the intensities is 95 + 95 = 190. In the middle at D = L/2, the sum of the intensities is 92.5 + 07.5 = 190. Thus, counter-winding fiber **12** compensates for the diminished amounts of radiated light **152'** generated along the length of the fiber for each pass and creates a relatively uniform (i.e., a substantially constant intensity) extended illumination source from light-source fiber portion **12B**.

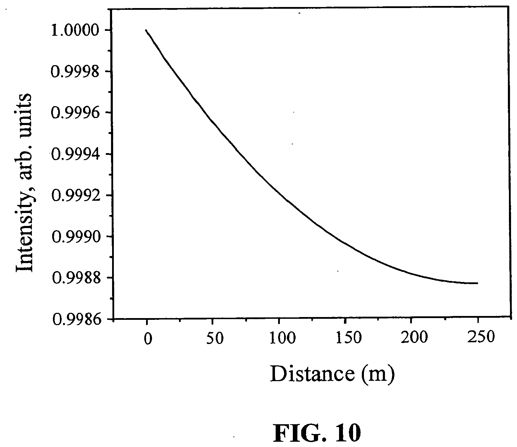

FIG. 10 is a plot of the relative intensity I vs. distance D for an example embodiment of light-source fiber portion **12B** of length **L** that includes a total of four counter-wound layers of fibers **12** in light-source fiber portion **12B** that create substantially uniform radiated light **152'** along the length of the light-source fiber portion. Note that over the length L = 250 meters, the intensity I of emitted radiation **152'** drops by less than 0.5%.

Because fiber **12** is bend-insensitive even in the visible wavelength band, forming relatively strong bends **130** that enhance scattering will not disturb the uniformity of the radiation along the length because of bending loss.

In order to create a uniform extended illumination from illumination system **100B**, example embodiments of the system include forming high-loss section **12B** by counter-winding **12** in opposite directions. Depending on attenuation and dimensions of bends **130**, the required length of the fiber can be determined.

In an example embodiment, light-source fiber portion **12B** emits radiated light **152'** having an intensity that preferably varies by no more that +/- 10%, more preferably by +/-5 %, even more preferably by +/- 2.5%, and even more preferably by +/- 1% along the length of the light source fiber portion.

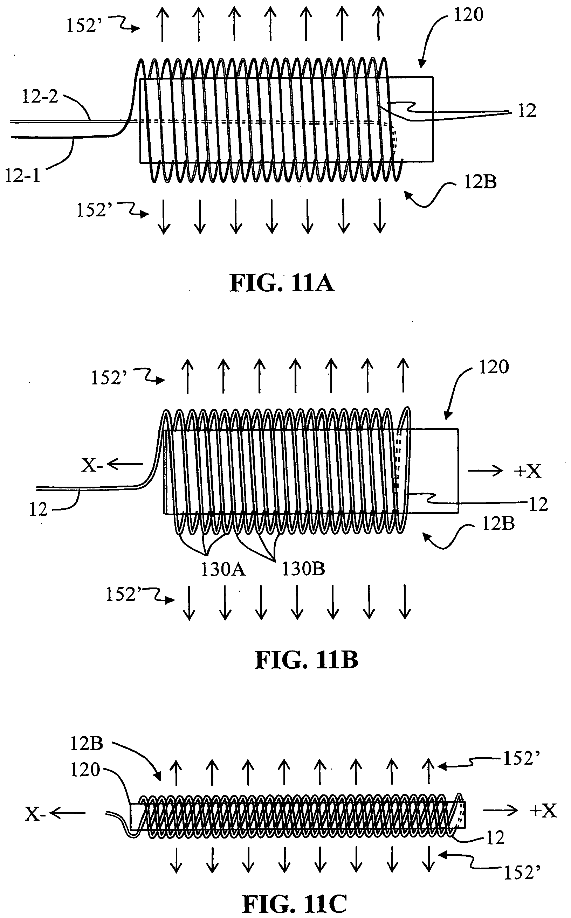

**FIG. 11A** is a close-up view of light-source fiber portion **12** of illumination system **100** illustrating an example embodiment that includes two counter-wound fibers **12** (identified as **12-1** and **12-2**) wound around support structure **120**. This embodiment serves to uniformize radiated light **152'** by countering the effect of decreased amounts of radiated light along the length of each fiber **12**. In other example embodiment, multiple fibers **12** are wound around support structure in a number of configurations such as shown in **FIG. 11A** to uniformize radiated light **152'**.

**FIG. 11B** is similar to **FIG. 11A**, except that the same fiber **12** is counter-wound in the +X and -X directions. Fiber **12** can be counter-wound a number of times, with the exact number limited mainly by the overall length of the fiber and the size of biological chamber interior **172**. Counter-winding fiber **12** serves to uniformize radiated light **152'** by making up for the reduction in the amount of radiated light along the length L of the fiber, such as illustrated in **FIG. 10**.

**FIG. 11C** is similar to **FIG. 11B**, illustrating an example embodiment of light-source fiber portion **12B** of fiber **12**, showing multiple counter-windings, wherein the counter-windings are relatively tight and wherein the counter-windings "cross-wound," i.e., the windings in the +X direction are angled in a direction opposite to that of the counter-windings in the -X direction. Such "cross-winding" serves to further improve the uniformity of radiated light **152'**.

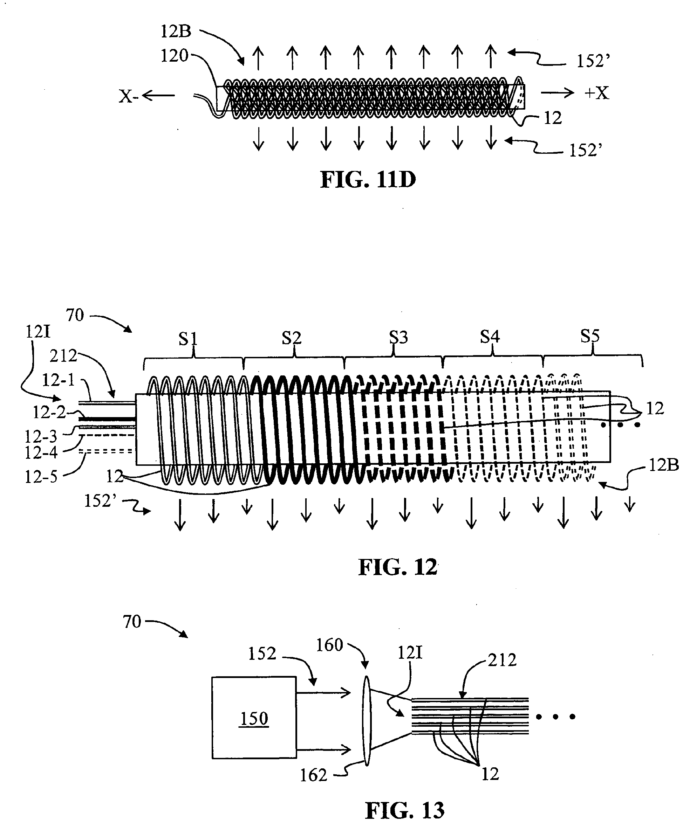

**FIG. 11D** is similar to **FIG. 11C**, and illustrates an example embodiment of the light-source fiber portion **12B** of the fiber **12** with even more counter-windings. Note that the radiation pattern of radiation **152'** is radially symmetric for the geometry of the light-source fiber portions **12B** illustrated in **FIGS. 11A-11D**.

In an example embodiment, light-source fiber portion **12B** of illumination system **100** according to **FIG.**

**11B** is formed using fiber **12** in the form of a multi-mode 125 um (radius) nanostructure fiber having an overall length of about 100 m wound around a support structure **120** in the form of a 1 cm diameter TYLON tube. The bend loss for this type of fiber is relatively moderate and slightly higher than the Rayleigh scattering loss, but the uniform counter-windings produce substantially uniform illumination.

**FIG. 12** illustrates an example embodiment of light-source fiber portion **12B** of illumination system **100** formed from sequential windings of multiple fibers (e.g., fiber fibers **12-1**, **12-2**, **12-3**, **12-4** and **12-5**). Fibers **12** are arranged in a fiber bundle **212** at their respective input ends **12I** and are configured to form sequential windings sections **S** (**S1** through **S5**) along the length of support structure **120** to form an extended light source. The sequential winding configuration of fibers **12** enhances the uniformity of radiated light **152'** by each section **S** serving as an extended light source that provides substantially uniform radiated light **152'**. The axial length of each section **S** is selected so that the amount of radiated light **152'** as a function of axial length does not fall below a certain threshold value (e.g., ~ 95%) of the inputted light at input end **12I**, such as illustrated in the plot of **FIG. 9**.

Subsequent sections **S** are formed, for example, by switching fiber **12-*n*** with the next fiber **12-(*n+1*)** that was an internal fiber.

For example, if there are 100 fibers **12** in a bundle supported by support structure **120**, and the desired length of the extended light source is 10 m, fiber **12-1** is wound around the bundle for the first 0.1 m in section **S1**, fiber **12-2** for the second 0.1 m in section **S2**, and so on until fiber **12-100** is wound around the bundle for the last 0.1 m in section **S100**. In an example embodiment, the sequential winding of different fibers **12** in light-source fiber portion **12B** is accomplished using an automated process similar to those used to strand optical fiber cables. In an example embodiment, one or more sections **S** include counter-wound fibers **12** that increase the uniformity of radiated light **152** from the one or more sections. The use of sequential windings allows for the formation of a lengthy extended light-source fiber portion **12B**.

**FIG. 13** illustrates an example embodiment of the front portion (i.e., the light generating and collecting portion) of illumination system **70** wherein light source **150** and optical coupling system **160** are configured to couple light **152** from the light source into the respective input ends **12I** of multiple fibers **12** arranged, for example, in a fiber bundle **212** as shown. Fiber bundle **212** is a convenient way to deliver concentrated sunlight to chamber interior **172**. Hundred of Watts of solar energy can be delivered to chamber interior **172** in this manner. In an alternative embodiment similar to that shown in **FIG. 13**, the various fibers **12** are optically coupled to one or more light sources **150** rather than to a single light source.

*Coatings*

In an example embodiment, fiber **12** in light-source fiber portion **12B** may include a layer **44** as discussed above in connection with **FIG. 2**. In an example embodiment, layer **44** comprises a hydrophilic coating such as a UV- cure acrylate coating that provides improved wet adhesion. A hydrophilic coating **44** serves as a cell growth medium as well as a protective covering for fiber **12**. As such, chemical modification or raw material substitution may be necessary to ensure that cell death does not occur.

Examples hydrophilic coatings for layer **44** are those commonly used for improving cell adhesion and growth to surfaces and contain carboxylic acid functionality and amine functionality (e.g. formulations containing acrylic acid or acrylamides). In addition, hydrophilic coatings for layer **44** may be enhanced by serving as a reservoir for nutrients essential for the growth of biological material **180**.

In an example embodiment, layer **44** includes fluorescent or ultraviolet absorbing molecules that serve to modify radiated light **152'** to produce light similar to that obtained with commercially available "grow lights."

The optical fiber(s), such as the nanostructured optical fiber(s) may be enclosed within a polymeric, metal, or glass covering (or coatings), wherein said covering has a minimum outer dimension (e.g., diameter) greater than 250 $\mu$m. If the fiber(s) has a metal coating, the metal coating may open sections, for modification of radiation in radial space (radially) or along the length of the fiber.

As stated above, the optical fiber may comprising a hydrophilic coating disposed on the optical fiber outer surface. Alternatively, a hydrophilic coating may be disposed on outer surface of the fiber ribbon. Also, fluorescent species (e.g., ultraviolet-absorbing material) may be disposed in the optical fiber coating.

Furthermore, an additional a coating layer may be provided on the fiber outer surface, this layer being configured to modify the radiated light. The additional coating may have scattering particles such as $TiO_2$ to modify light distribution in radial space. The coating may include fluorescence species to modify emitted wavelength of the light or may have absorbing species such as ink to additionally modify emmited light spectrum.

*Example illumination system configurations*

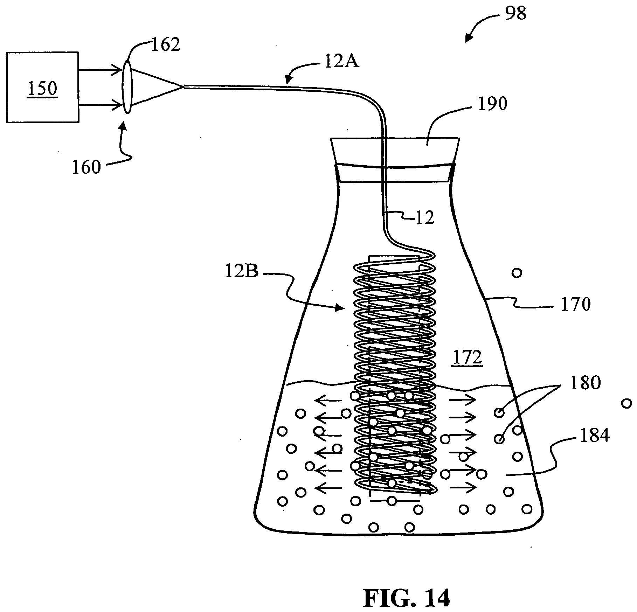

**FIG. 14** illustrates an example embodiment of a biological growth system **98** and an illumination system **100** as used in the biological growth system, wherein biological chamber **170** is in the form of a flask. In the embodiment of **FIG. 14**, light-source fiber portion **12B** is formed from a single counter-wound fiber similar to that shown in **FIG. 11B**. Illumination system **100** of **FIG. 14** was used to conduct the biological growth experiments described below.

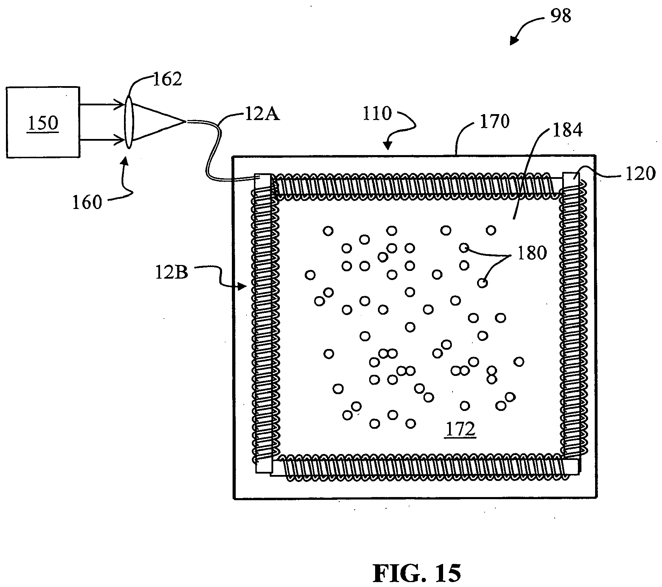

**FIG. 15** illustrates an example embodiment (top-down view) of a biological growth system **98** and an illumination system **100** as used in the biological growth system, wherein the light-source fiber portion **12B** of illumination system **100** has a rectangular configuration suitable for use in the rectangular-cross-section biological chamber **110** as shown.

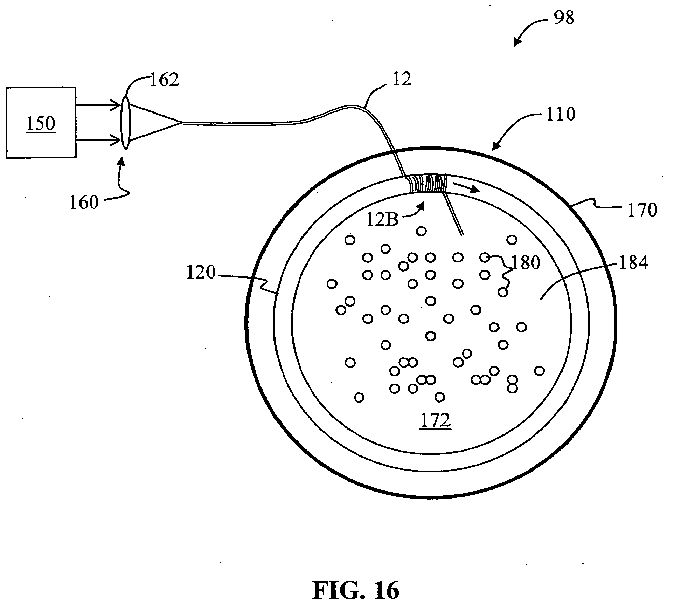

**FIG. 16** illustrates an example embodiment (top down view) of a biological growth system **98** and an illumination system **100** as used in the biological growth system, wherein illumination system **100** has a light-source fiber portion **12B** configured in a circular geometry for use in a round-cross-section biological chamber **110** as shown. An example embodiment of illumination system **100** of **FIG. 16** utilizes a ring-type support structure **120** as shown. **FIG. 16** shows fiber **12** in the process of being wound clockwise around support structure **120** to form the circular light-source fiber portion **12B**.

Other shapes and geometries for light-source fiber portion **12B** of illumination system **100** are encompassed by the present invention and are a function of the particular needs of the application and in particular the geometry of biological chamber **110**. In an example embodiment, light-source fiber portion **12B** is defined by the geometry of biological chamber **110**. For example, the design of light-source fiber portion **12B** may be defined by the need to provide substantially uniform exposure throughout all parts of the chamber.

An additional advantage of the light diffusing fiber is the proven ability to bundle the fibers into multi-fiber collections known as ribbons. Typical ribbon structures range from 2-36 fibers. Another advantage derived from the ribbon structure is the need for winding of fibers no longer is a necessity as ribbons may be made to form other bent structures such as waves, helices, or spirals thereby allowing light to scatter. Furthermore, the use of multi-fiber ribbons affords the possibility of having large stacks of ribbons. Such ribbon stacks would allow not only a more concentrated mass of light delivery, but also open the possibility to the use of different light sources, such as red lasers, sunlight, light emitting diodes, or guidance of point light sources. For example, according to one embodiment, a plurality of nanostructured optical fibers may be wound around a support structure, and each nanostructured optical fiber may be optically coupled to either the light source or a plurality of light sources, while the nanostructured optical fibers are bundled together in at least one of: a ribbon, ribbon stack, or a round bundle.

*Experimental Results*

Experiments were conducted wherein illumination system **100** having a light-source fiber portion **12B** in the form illustrated in **FIG. 14** was used to support the growth of biological material in the form of cyanobacteria (*Synechocystis sp.* PCC 6803). The experiment included a control group that did not use illumination system **100** and instead used a fluorescent light source that provided the same amount of light as illumination system **100**.

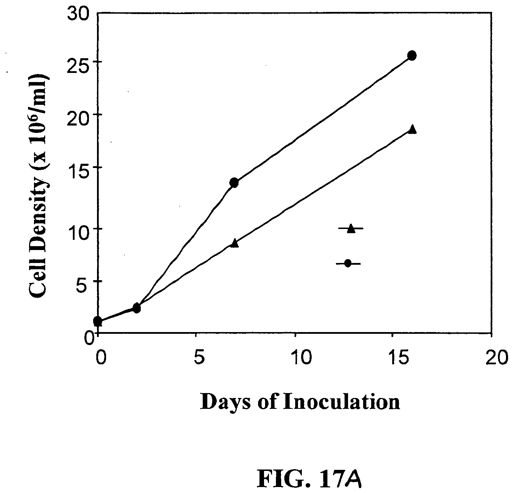

**FIG. 17A** is a plot of biomass (expressed as cell density) vs. days of inoculation growth for both the illuminated cyanobacteria ("test group") and the control group. The plot shows a higher growth rate for the test group as compared to the control group under the current experimental setting. Both test and control groups were placed inside of a biological chamber **110** in the form of a flask (shaker). Except for the illumination system **100**, the growth conditions were the same for both control and test groups, namely: 30°C, aeration speed of 105 rpm, and about 0.03% $CO_2$ (ambient air).

For the control group, the illumination was provided by fluorescent lamps above the growth chamber so the light intensity inside chamber was 50 $\mu$mol/m$^2$/s. For the test group, a laser light source that generated light at 530 nm was used.

Light-source fiber portion **12B** was placed inside the culture medium (i.e., the biological material) and the flask was covered by aluminium foil. The intensity of emitted radiation **152'** from light-source fiber portion **12B** was adjusted to have the same intensity as the control group. Light-source fiber portion **12B** demonstrated very good biocompatibility for supporting cyanobacteria growth, and the experiments support the position that illumination system **100** would be well-suited for other types of biological applications and different biological chamber geometries.

Additional experiments were conducted wherein illumination system **100** having a light-source fiber portion **12B** in the form illustrated in **FIG. 14** was used to support the growth of biological material in the form of cyanobacteria (*Synechocystis sp.* PCC 6803). The experiment included a control group that did not use illumination system **100** and instead used a light source delivering light through a fiber tip (point source) that provided the same amount of light as illumination system **100**. Exemplary light sources may be lasers, light-emitting diodes, sunlight or fluorescent light sources.

**FIG. 17B** is a plot of biomass (expressed as cell density) vs. days of inoculation growth for both the illuminated cyanobacteria ("test group") and the control group. The plot shows a higher growth rate for the test group as compared to the control group under the current experimental setting. Both test and control groups were placed inside of a biological chamber **110** in the form of a flask (shaker). Except for the illumination system **100**, the growth conditions were the same for both control and test groups, namely: 30°C, aeration speed of 105 rpm, and about 0.03% $CO_2$ (ambient air). A laser light source that generated light at 530 nm was used for both the control and the test group.

Light-source fiber portion **12B** was placed inside the culture medium (i.e., the biological material) and the flask was covered by aluminium foil. The intensity of emitted radiation **152'** from light-source fiber portion **12B** was adjusted to have the same intensity as the control group. Light-source fiber portion **12B** demonstrated very good biocompatibility for supporting cyanobacteria growth, and the experiments support the position that illumination system **100** would be well-suited for other types of biological applications and different biological chamber geometries.

It is to be understood that the foregoing description is exemplary of the invention only and is intended to provide an overview for the understanding of the nature and character of the invention as it is defined by the claims. The accompanying drawings are included to provide a further understanding of the invention and are incorporated and constitute part of this specification. The drawings illustrate various features and embodiments of the invention which, together with their description, serve to explain the principals and operation of the invention.

**Claims**

1. An illumination system for a biological growth system having a biological chamber with an interior configured to contain biological material, comprising:

   a light source that generates light having a wavelength to which the biological material is sensitive; and
   at least one nanostructured optical fiber having a central axis, a silica based glass core, a glass cladding structure comprising the nanostructures, an outer surface and an end optically coupled to the light source, the fiber configured to have a plurality of bends formed therein so as to scatter guided light away from the central axis and through the outer surface to form a light-source fiber portion having a length that emits substantially uniform radiation over its length.

2. The illumination system of claim 1, wherein the at least one nanostructured optical fiber is counter-wound about a support structure.

3. The illumination system of claim 1, wherein the plurality of bends induce Rayleigh scattering.

4. The illumination system of claim 1, wherein the substantially uniform radiation varies by no more than +/- 10% over the light-source fiber portion.

5. The illumination system of claim 1, further including at least one optical fiber section optically coupled at a first end to the at least one nanostructure optical fiber and at a second end to the light source.

6. The illumination system of claim 1, further comprising: (i) a hydrophilic coating disposed on the optical fiber outer surface; and/or (ii) fluorescent and/or ultraviolet-absorbing molecules disposed on the optical fiber outer surface.

7. A biological growth system comprising:

   a biological chamber with an interior configured to contain biological material;
   a light source that generates light having a wavelength to which the biological material is sensitive; and
   at least one nanostructured optical fiber having a central axis, a silica based glass core, a glass cladding structure comprising the nanostructures, an outer surface and an end optically coupled to the light source, the fiber configured to have a plurality of bends formed therein so as to scatter guided light away from the central axis and through the outer surface to form a light-source fiber portion having a length that emits substantially uniform radiation over its length.

8. The biological growth system of claim 7, wherein the at least one nanostructured fiber is supported by a support structure.

9. The biological growth system of claim 8, wherein the at least one nanostructured fiber is counter-wound around the support structure one or more times so that the substantially uniform radiation varies in uniformity by no more than +/- 10% over the light-source fiber portion.

10. The biological growth system of claim 8, wherein a plurality of nanostructured optical fibers are wound around the support structure in sequence along the support structure, with each optical fiber optically coupled either to the light source or one or more light sources.

11. A method of providing substantially uniform illumination to a biological chamber having an interior configured to support biological material, comprising:

    in at least one nanostructured optical fiber having a silica based glass core, a glass cladding structure having randomly arranged voids with diameters between $1\times10^{-6}$ m and $1\times10^{-5}$ m, a central axis and an outer surface, forming a plurality of bends configured to substantially increase Rayleigh scattering in the at least one fiber, the plurality of bends forming a light-source fiber portion of the at least one fiber;
    disposing the light-source fiber portion in the biological chamber interior; and
    inputting light into the at least one fiber and Rayleigh-scattering a portion of said light away from the central axis and through the outer surface, thereby emitting substantially uniform radiation from the light-source fiber portion, wherein said substantially uniform radiation includes a wavelength to which the biological material is sensitive.

12. The method of claim 11, including counter-winding the at least one fiber one or more times so that the substantially uniform radiation varies in uniformity by no more than +/- 10% over the light-source fiber portion.

13. The method of claim 12, wherein the biological chamber interior has a geometry, the method comprising situating said fiber inside said chamber and forming the light-source fiber portion to correspond to said geometry.

14. The method of claim 11, further including providing a layer on the fiber outside surface, said layer configured to modify the radiated light.

15. The method of claim 14, wherein said layer includes fluorescent and/or ultraviolet-absorbing molecules.

**Patentansprüche**

1. Beleuchtungssystem für ein biologisches Wachstumssystem, das eine biologische Kammer mit einem Innenraum aufweist, der dazu ausgelegt ist, ein biologisches Material zu enthalten, umfassend:

   eine Lichtquelle, die Licht erzeugt, das eine Wellenlänge aufweist, auf die das biologische Material empfindlich ist; und
   mindestens eine nanostrukturierte Lichtleitfaser, die eine Mittelachse, einen Glaskern auf Kieselerdebasis, eine Verkleidungskonstruktion aus Glas, die die Nanostrukturen umfasst, eine äußere Oberfläche und ein Ende, das optisch mit der Lichtquelle gekoppelt ist, aufweist, wobei die Faser dazu ausgelegt ist, eine Vielzahl von darin gebildeten Biegungen aufzuweisen, um geführtes Licht von der Mittelachse weg und durch die äußere Oberfläche zu streuen, um einen Lichtquellenfaseranteil zu bilden, der eine Länge aufweist, der im Wesentlichen einheitliche Strahlung über seine Länge abgibt.

2. Beleuchtungssystem nach Anspruch 1, wobei die mindestens eine nanostrukturierte Optikfaser entgegengesetzt um eine Stützstruktur gewickelt ist.

3. Beleuchtungssystem nach Anspruch 1, wobei die Vielzahl von Biegungen Rayleigh-Streuung hervorruft.

4. Beleuchtungssystem nach Anspruch 1, wobei die im Wesentlichen einheitliche Strahlung um nicht mehr als +/- 10 % über den Lichtquellenfaseranteil variiert.

**5.** Beleuchtungssystem nach Anspruch 1, ferner enthaltend mindestens einen optischen Faserabschnitt, der optisch an einem ersten Ende an die mindestens eine nanostrukturierte Optikfaser und an einem zweiten Ende an die Lichtquelle gekoppelt ist.

**6.** Beleuchtungssystem nach Anspruch 1, ferner umfassend: (i) eine hydrophile Beschichtung, die an der Optikfaseraußenfläche angeordnet ist; und/oder (ii) fluoreszierende und/oder Ultraviolett absorbierende Moleküle, die an der Optikfaseraußenfläche angeordnet sind.

**7.** Biologisches Wachstumssystem, umfassend:

eine biologische Kammer mit einem Innenraum, der dazu ausgelegt ist, ein biologisches Material zu enthalten;
eine Lichtquelle, die Licht erzeugt, das eine Wellenlänge aufweist, auf die das biologische Material empfindlich ist; und
mindestens eine nanostrukturierte Lichtleitfaser, die eine Mittelachse, einen Glaskern auf Kieselerdebasis, eine Verkleidungskonstruktion aus Glas, die die Nanostrukturen umfasst, eine äußere Oberfläche und ein Ende, das optisch mit der Lichtquelle gekoppelt ist, aufweist, wobei die Faser dazu ausgelegt ist, eine Vielzahl von darin gebildeten Biegungen aufzuweisen, um geführtes Licht von der Mittelachse weg und durch die äußere Oberfläche zu streuen, um einen Lichtquellenfaseranteil zu bilden, der eine Länge aufweist, der im Wesentlichen einheitliche Strahlung über seine Länge abgibt.

**8.** Biologisches Wachstumssystem nach Anspruch 7, wobei die mindestens eine nanostrukturierte Faser durch eine Stützstruktur gestützt ist.

**9.** Biologisches Wachstumssystem nach Anspruch 8, wobei die mindestens eine nanostrukturierte Faser ein oder mehrere Male entgegengesetzt um die Stützstruktur gewickelt ist, sodass die im Wesentlichen einheitliche Strahlung in Einheitlichkeit um nicht mehr als +/- 10 % über den Lichtquellenfaseranteil variiert.

**10.** Biologisches Wachstumssystem nach Anspruch 8, wobei eine Vielzahl von nanostrukturierten Optikfasern um die Stützstruktur in Folge entlang der Stützstruktur gewickelt ist, wobei jede Optikfaser optisch entweder mit der Lichtquelle oder einer oder mehreren Lichtquellen gekoppelt ist.

**11.** Verfahren zum Bereitstellen im Wesentlichen einheitlicher Beleuchtung für eine biologische Kammer, die einen Innenraum aufweist, der dazu ausgelegt ist, biologisches Material zu stützen, umfassend:

in mindestens einer nanostrukturierten Optikfaser Aufweisen eines Glaskerns auf Kieselerdebasis, einer Verkleidungskonstruktion aus Glas, die zufällig angeordnete Hohlräume mit Durchmessern zwischen $1 \times 10^{-6}$ m und $1 \times 10^{-5}$ m, eine Mittelachse und eine äußere Oberfläche aufweist, die eine Vielzahl von Biegungen bildet, die dazu ausgelegt sind, im Wesentlichen Rayleigh-Streuung in der mindestens einen Faser zu erhöhen, wobei die Vielzahl von Biegungen einen Lichtquellenfaseranteil der mindestens einen Faser bildet;
Anordnen des Lichtquellenfaseranteils in dem Innenraum der biologischen Kammer; und
Eingeben von Licht in die mindestens eine Faser und Rayleigh-Streuen eines Abschnitts des Lichts weg von der Mittelachse und durch die äußere Oberfläche, dadurch Abgeben im Wesentlichen einheitlicher Strahlung von dem Lichtquellenfaseranteil, wobei die im Wesentlichen einheitliche Strahlung eine Wellenlänge enthält, auf die das biologische Material empfindlich ist.

**12.** Verfahren nach Anspruch 11, enthaltend Entgegenwickeln der mindestens einen Faser ein oder mehrere Male, sodass die im Wesentlichen einheitliche Strahlung in Einheitlichkeit um nicht mehr als +/- 10 % über den Lichtquellenfaseranteil variiert.

**13.** Verfahren nach Anspruch 12, wobei der Innenraum der biologischen Kammer eine Geometrie aufweist, wobei das Verfahren Platzieren der Faser innerhalb der Kammer und Bilden des Lichtquellenfaseranteils umfasst, um der Geometrie zu entsprechen.

**14.** Verfahren nach Anspruch 11, ferner enthaltend Bereitstellen einer Schicht auf der Faseraußenfläche, wobei die Schicht dazu ausgelegt ist, das abgestrahlte Licht zu ändern.

**15.** Verfahren nach Anspruch 14, wobei die Schicht fluoreszierende und/oder Ultraviolett absorbierende Moleküle enthält.

**Revendications**

**1.** Système d'éclairement destiné un système de croissance biologique comportant une chambre biologique dont l'intérieur est conçu pour contenir une substance biologique, comprenant :

une source de lumière qui génère de la lumière ayant une longueur d'onde à laquelle est sensible la substance biologique ; et
au moins une fibre optique nanostructurée ayant un axe central, comportant un coeur en verre à base de silice, une structure de gainage en verre comprenant les nanostructures, une surface extérieure et une extrémité couplée optiquement à la source de lumière, la fibre étant conçue pour comporter plusieurs courbures formées dans cette dernière de façon à diffuser de la lumière guidée à l'écart de l'axe central et à travers la surface extérieure de façon à former une partie de fibre source de lumière ayant une longueur qui émet un rayonnement sensiblement uniforme sur sa longueur.

2. Système d'éclairement selon la revendication 1, dans lequel l'au moins une fibre optique nanostructurée est contre-enroulée autour d'une structure de support.

3. Système d'éclairement selon la revendication 1, dans lequel les plusieurs courbures induisent une diffusion de Rayleigh.

4. Système d'éclairement selon la revendication 1, dans lequel la variation de rayonnement sensiblement uniforme est inférieure ou égale à +/- 10 % sur la partie de fibre source de lumière.

5. Système d'éclairement selon la revendication 1, comprenant en outre au moins une section de fibre optique couplée optiquement, au niveau d'une première extrémité, à l'au moins une fibre optique nanostructurée et, au niveau d'une seconde extrémité, à la source de lumière.

6. Système d'éclairement selon la revendication 1, comprenant en outre : (i) un revêtement hydrophile disposé sur la surface extérieure de fibre optique ; et/ou (ii) des molécules fluorescentes et/ou absorbant la lumière ultraviolette disposées sur la surface extérieure de fibre optique.

7. Système de croissance biologique, comprenant :

une chambre biologique dont l'intérieur est conçu pour contenir une substance biologique ; une source de lumière qui génère de la lumière ayant une longueur d'onde à laquelle est sensible la substance biologique ; et au moins une fibre optique nanostructurée ayant un axe central, comportant un coeur en verre à base de silice, une structure de gainage en verre comprenant les nanostructures, une surface extérieure et une extrémité couplée optiquement à la source de lumière, la fibre étant conçue pour comporter plusieurs courbures formées dans cette dernière de façon à diffuser de la lumière guidée à l'écart de l'axe central et à travers la surface extérieure de façon à former une partie de fibre source de lumière ayant une longueur qui émet un rayonnement sensiblement uniforme sur sa longueur.

8. Système de croissance biologique selon la revendication 7, dans lequel l'au moins une fibre nanostructurée est supportée par une structure de support.

9. Système de croissance biologique selon la revendication 8, dans lequel l'au moins une fibre nanostructurée est contre-enroulée, une ou plusieurs fois, autour de la structure de support de sorte que la variation d'uniformité de rayonnement sensiblement uniforme soit inférieure ou égale à +/-10 % sur la partie de fibre source de lumière.

10. Système de croissance biologique selon la revendication 8, dans lequel plusieurs fibres optiques nanostructurées sont enroulées autour de la structure de support en séquence le long de la structure de support, chaque fibre optique étant optiquement couplée soit à la source de lumière soit à une ou plusieurs sources de lumière.

11. Procédé de fourniture d'un éclairement sensiblement uniforme à une chambre biologique dont l'intérieur est conçu pour supporter une substance biologique, comprenant :

dans au moins une fibre optique nanostructurée comportant un coeur en verre à base de silice, une structure de gainage en verre comportant des vides agencés de façon aléatoire ayant des diamètres compris entre $1\times10^{-6}$ m et $1\times10^{-5}$ m, un axe central et une surface extérieure, la formation de plusieurs courbures conçues pour augmenter sensiblement la diffusion de Rayleigh dans l'au moins une fibre, les plusieurs courbures formant une partie de fibre source de lumière de l'au moins une fibre ; la disposition de la partie de fibre source de lumière à l'intérieur de la chambre biologique ; et l'entrée de lumière dans l'au moins une fibre et la diffusion de Rayleigh d'une partie de ladite lumière à l'écart de l'axe central et à travers la surface extérieure, ce qui permet d'émettre un rayonnement sensiblement uniforme depuis la partie de fibre source de lumière, dans lequel ledit rayonnement sensiblement uniforme a une longueur d'onde à laquelle est sensible la substance biologique.

12. Procédé selon la revendication 11, comprenant le contre-enroulement, une ou plusieurs fois, de l'au moins une fibre de sorte que la variation d'uniformité de rayonnement sensiblement uniforme soit inférieure ou égale à +/- 10 % sur la partie de fibre source de lumière.

**13.** Procédé selon la revendication 12, dans lequel l'intérieur de la chambre biologique a une certaine géométrie, le procédé comprenant la mise en place de ladite fibre à l'intérieur de ladite chambre et la formation de la partie de fibre source de lumière pour qu'elle corresponde à ladite géométrie.

**14.** Procédé selon la revendication 11, comprenant en outre l'application d'une couche à la surface extérieure de fibre, ladite couche étant conçue pour modifier la lumière rayonnée.

**15.** Procédé selon la revendication 14, dans lequel ladite couche comprend des molécules fluorescentes et/ou absorbant la lumière ultraviolette.

**FIG. 1**

**FIG. 2**

**FIG. 3A**

**FIG. 3B**

**FIG. 4**

**FIG. 5**

**FIG. 6**

**FIG. 8**

**FIG. 7**

**FIG. 9**

FIG. 10

**FIG. 11A**

**FIG. 11B**

**FIG. 11C**

**FIG. 11D**

**FIG. 12**

**FIG. 13**

**FIG. 14**

**FIG. 15**

**FIG. 16**

**FIG. 17A**

**FIG. 17B**

**FIG. 18**

## REFERENCES CITED IN THE DESCRIPTION

**Patent documents cited in the description**

- DE 4416069
- US 20070104437 A
- US 20050137657 A
- US 583098 A